# EUROPÄISCHE PATENTSCHRIFT

(11) **EP 1 043 955 B1**
(45) Veröffentlichungstag und Bekanntmachung des Hinweises auf die Patenterteilung: **24.10.2001**
(21) Anmeldenummer: 99934582.0
(22) Anmeldetag: 06.07.1999
(51) Int. Cl.: A61B 17/28

(54) **MEDIZINISCHE ZANGE MIT ZWEI UNABHÄNGIG VONEINANDER BEWEGLICHEN MAULTEILEN**
MEDICAL FORCEPS WITH TWO INDEPENDENTLY MOBILE JAW PARTS
PINCE MEDICALE COMPORTANT DEUX ELEMENTS A MACHOIRES DEPLACABLES INDEPENDAMMENT L'UN DE L'AUTRE

(30) Priorität: 25.07.1998 DE 19833600
(43) Veröffentlichungstag der Anmeldung: 18.10.2000
(73) Patentinhaber: Karl Storz GmbH & Co. KG, 78532 Tuttlingen (DE)
(72) Erfinder: BACHER, Uwe, D-78532 Tuttlingen (DE)
(74) Vertreter: Heuckeroth, Volker
(86) Internationale Anmeldenummer: EP9904685
(87) Internationale Veröffentlichungsnummer: WO0006033

(56) Entgegenhaltungen:
- EP-A- 0 640 319
- DE-U- 9 409 979
- US-A- 5 171 257

## Beschreibung

Die Erfindung betrifft eine medizinische Zange, mit zwei unabhängig voneinander beweglichen Maulteilen am distalen Ende, mit einem Schaft, in dem sich zwei unabhängig voneinander axial verschiebbare, nebeneinander angeordnete Stangen längs erstrecken, wobei die erste Stange mit dem ersten Maulteil und die zweite Stange mit dem zweiten Maulteil kraftschlüssig verbunden ist, und mit einer Handhabe am proximalen Ende, die zwei Betätigungselemente aufweist, wobei das erste Betätigungselement mit der ersten Stange und das zweite Betätigungselement mit der zweiten Stange kraftschlüssig verbunden ist, wobei die erste Stange und die zweite Stange an ihrem proximalen Ende jeweils ein Verbindungselement und das erste Betätigungselement und das zweite Betätigungselement an ihrem distalen Ende jeweils ein dazu im wesentlichen komplementär ausgebildetes Verbindungselement aufweisen.

Eine derartige Zange ist aus der US-A-5 171 257 bekannt.

Zangen der eingangs genannten Art werden in der endoskopischen Chirurgie üblicherweise als Multifunktionsinstrumente eingesetzt, beispielsweise um Gewebe mit dem einen Maulteil zu schneiden und mit dem anderen Maulteil zur Entnahme des Gewebes aus dem Körper zu fassen. Dementsprechend ist das eine Maulteil als Schneidwerkzeug ausgebildet, während das andere Maulteil als Faßwerkzeug ausgestaltet ist. Beide Maulteile sind unabhängig voneinander beweglich, d.h. sie können unabhängig voneinander geschlossen und geöffnet werden. Dazu weist die Zange für jedes Maulteil ein Betätigungselement auf, mit dem das entsprechende Maulteil geöffnet bzw. geschlossen werden kann.

Zur Kraftübertragung von den Betätigungselementen auf die Maulteile sind zwei längs nebeneinander angeordnete Stangen in dem Schaft angeordnet, von denen die erste Stange mit ihrem proximalen Ende mit dem ersten Betätigungselement und mit ihrem distalen Ende mit dem ersten Maulteil, und die zweite Stange mit ihrem proximalen Ende mit dem zweiten Betätigungselement und mit ihrem distalen Ende mit dem zweiten Maulteil kraftschlüssig verbunden ist. Die beiden Stangen arbeiten zum Schließen und Öffnen der Maulteile auf Zug bzw. Druck.

Bei der eingangs genannten bekannten Zange ist nachteilig, daß sich die Stangen und der Schaft nicht, zumindest nicht auf einfach handhabbare Weise von der Handhabe abnehmen lassen, wodurch diese Zange nicht oder nur erschwert einer gründlichen Reinigung zugänglich ist. Bei einem Einsatz einer solchen Zange in einem operativen Eingriff sammeln sich nämlich Verunreinigungen zwischen dem Schaft und den Stangen an. Bei der bekannten Zange ist zwischen der Handhabe und dem Schaft eine Spülöffnung vorgesehen, durch die Spülflüssigkeit in den Schaft zur Reinigung des Innenraumes des Schaftes eingeleitet werden kann. Durch das Einleiten einer Reinigungsflüssigkeit ist jedoch eine gründliche Reinigung des Innenraumes des Schaftes und der sich darin erstreckenden Stangen nicht gewährleistet, weil beispielsweise in den Schaft eingedrungene Gewebestücke zwischen den nebeneinander liegenden Stangen oder an Kanten und in Nischen haften bleiben können, die sich durch die Spülflüssigkeit nicht entfernen lassen.

Eine weitere, nicht zerlegbare Zange mit zwei unabhängig voneinander beweglichen Maulteilen ist aus der US-A-5 403 332 bekannt.

Der Erfindung liegt daher die Aufgabe zugrunde, eine medizinische Zange der eingangs genannten Art dahingehend weiterzubilden, daß die Zange zur Reinigung zerlegbar ist, wobei zumindest die Stangen von der Handhabe abnehmbar sind, und wobei die Zange nach dem Reinigen für einen erneuten Einsatz schnell und auf einfach zu handhabende Weise zusammenbaubar sein soll.

Erfindungsgemäß wird diese Aufgabe hinsichtlich der eingangs genannten medizinischen Zange dadurch gelöst, daß die Verbindungselemente der Stangen mit den Verbindungselementen der Betätigungselemente durch axiales Zusammenschieben der Stangen und der Betätigungselemente und/oder durch eine gemeinsame Verschwenkung der beiden Stangen um eine Längsachse kraftschlüssig, jedoch lösbar in Eingriff bringbar sind.

Bei der erfindungsgemäßen medizinischen Zange ist demnach vorgesehen, die Stangen von der Handhabe abnehmbar auszugestalten, wozu die proximalen Enden der Stangen und die distalen Enden der Betätigungselemente zueinander im wesentlichen komplementäre Verbindungselemente aufweisen, die voneinander lösbar sind. Der Verbindungsmechanismus der erfindungsgemäßen Zange zeichnet sich durch seine konstruktiv einfache Ausgestaltung und durch seine einfach zu handhabende Bedienung aus, weil die Verbindung der Stangen mit den Betätigungselementen durch einfache Manipulationen, nämlich durch Zusammenschieben und/oder durch Verschwenken der Stangen bewerkstelligt wird. Dieser Verbindungsmechanismus ist auch deswegen vorteilhaft, weil zum Verbinden der Stangen mit den Betätigungselementen keine Befestigungsmittel wie beispielsweise Schrauben und somit auch keine Werkzeuge wie Schraubendreher erforderlich sind, um die erfindungsgemäße Zange für einen erneuten Einsatz zusammenzusetzen. Axiales Zusammenschieben und/oder Verschwenken sind denkbar einfache Manipulationen. Die erfindungsgemäße medizinische Zange läßt sich nach dem Abnehmen der Stangen gründlich reinigen, weil die abgenommenen und damit freigelegten Stangen separat gewaschen werden können, so daß auch sich in Zwischenräumen zwischen den Stangen oder an Ecken ansammelnde Verunreinigungen sicher entfernt werden können. Die Verbindungselemente können komplementäre runde, bspw. kugelförmige und entsprechend kugelkalottenförmige Strukturen, oder nicht-runde Strukturen aufweisen, bspw. keilförmige Strukturen, die sich gegenseitig hintergreifende Abschnitte aufweisen, so daß Zug- und Druckkräfte von den Betätigungselementen auf die Stangen übertragen werden können. Die Stangen selbst können von jeder geeigneten Querschnittsform sein, bspw. rund oder nicht-rund, wie bspw. rechteckig.

Somit wird die der Erfindung zugrunde liegende Aufgabe vollkommen gelöst.

In einer bevorzugten Ausgestaltung der erfindungsgemäßen Zange sind die Verbindungselemente der Stangen und die Verbindungselemente der Betätigungselemente derart ausgebildet, daß sie durch eine Verschwenkung der Stangen um die vorgenannte Längsachse außer Eingriff bringbar sind.

Diese Maßnahme hat nun den weiteren Vorteil, daß sich die Stangen von den Betätigungselementen auf ebenso einfach zu handhabende Weise lösen lassen, ohne das dazu Werkzeuge oder dergleichen benutzt werden müssen. Der weitere Vorteil des durch Verschwenken der Stangen lösbaren Verbindungsmechanismus besteht in einem konstruktiv besonders einfachen Aufbau der erfindungsgemäßen Zange.

In einer weiteren bevorzugten Ausgestaltung kommen die Verbindungselemente der Stangen mit den Verbindungselementen der Betätigungselemente durch Betätigen der Betätigungselemente und/oder durch Betätigen der Maulteile rastend in Eingriff.

Hierbei ist weiterhin von Vorteil, daß das kraftschlüssige Verbinden der Stangen und der Betätigungselemente durch Betätigen der Betätigungselemente über an der Handhabe üblicherweise vorgesehene Griffelemente der Handhabe, die mit den Betätigungselementen in Verbindung stehen, bewerkstelligt wird, wodurch das Verbinden der Betätigungselemente mit den Stangen besonders einfach und sicher zu bewerkstelligen ist. Durch Betätigen der Btätigungselemente werden diese relativ zu den Stangen relativ verschoben, durch Betätigen der Maulteile werden die mit diesen verbundenen Stangen relativ zu den Betätigungselementen verschoben.

In einer weiteren bevorzugten Ausgestaltung weisen die Verbindungselemente der Stangen und/oder die Verbindungselemente der Betätigungselemente Anlaufschrägen auf.

Diese Maßnahme hat den weiteren Vorteil, daß die Verbindungselemente der Stangen und die Verbindungselemente der Betätigungselemente beim axialen Zusammenschieben eine Zwangsführung erfahren, durch die die Verbindungselemente der Stangen mit den Verbindungselementen der Betätigungselemente beim Zusammenschieben selbsttätig, und ohne das zuvor eine Raste oder dergleichen betätigt werden müßte, in Eingriff kommen bzw. ineinander einrücken.

Dabei ist es bevorzugt, wenn die Stangen an ihrem proximalen Ende geringfügig elastisch federnd verbiegsam sind.

Dies ist von Vorteil, weil die Stangen durch diese Ausgestaltung einerseits genügend nachgiebig sind, so daß die Verbindungselemente ohne erhöhten Reibungswiderstand an den Anlaufschrägen entlang gleiten können, indem die proximalen Enden der Stangen aus ihrer Längsachse ausgelenkt werden, und weil die Verbindungselemente der Stangen nach Überwinden der Anlaufschräge andererseits elastisch federnd mit den Verbindungselementen der Betätigungselemente rastend in Eingriff kommen und dadurch die kraftschlüssige Verbindung zwischen den Stangen und den Betätigungselementen herstellen.

In einer weiteren bevorzugten Ausgestaltung sind die Stangen gegen unerwünschtes Verschwenken gesperrt, wenn die Verbindungselemente der Stangen mit den Verbindungselementen der Betätigungselemente in Eingriff stehen.

Diese Maßnahme ist insbesondere mit der zuvor erwähnten Ausgestaltung von Vorteil, bei der die Verbindungselemente der Stangen mit den Verbindungselementen der Betätigungselemente durch eine Verschwenkung der Stangen um die Längsachse außer Eingriff bringbar sind, weil dadurch vermieden wird, daß sich die Stangen von den Betätigungselementen während eines Einsatzes der Zange bei einem operativen Eingriff lösen, wodurch die Betriebssicherheit der Zange verbessert ist.

In einer weiteren bevorzugten Ausgestaltung ist der Schaft abnehmbar mit der Handhabe verbunden.

Durch diese Maßnahme wird der weitere Vorteil erzielt, daß nicht nur die Stangen von der Handhabe abnehmbar und somit separat reinigbar sind, sondern auch der Schaft, so daß dieser ebenfalls, insbesondere innen gründlich gereinigt werden kann.

Dabei ist bevorzugt, wenn der Schaft mittels eines an der Handhabe angeordneten Rastmechanismus mit der Handhabe verbunden ist.

Diese Maßnahme hat den Vorteil, daß auch der Schaft auf einfach zu bedienende Weise mit der Handhabe verbindbar und von dieser abnehmbar ist, wobei der Rastmechanismus im Betrieb der Zange einen sicheren Zusammenhalt zwischen Handhabe und Schaft gewährleistet.

In einer weiteren bevorzugten Ausgestaltung sperrt der Rastmechanismus den Schaft gegen Verdrehen.

Diese Maßnahme hat den weiteren Vorteil, daß der Schaft ohne weitere konstruktive Maßnahmen gegen Verdrehen gesichert ist, sobald der Schaft mit der Handhabe verrastet ist, wodurch weitere Teile für eine Verdrehsicherung eingespart werden.

In einer weiteren bevorzugten Ausgestaltung ist der Schaft von den Stangen abnehmbar, und sind die Stangen mit dem Schaft im montierten Zustand drehfest verbunden.

Diese Maßnahme hat den Vorteil, daß die erfindungsgemäße Zange noch weiter zerlegbar ist, so daß die Reinigungseigenschaften der erfindungsgemäßen Zange weiter verbessert sind. Der weitere Vorteil dieser Maßnahme besteht darin, daß die Stangen bereits über den Schaft, mit dem sie im montierten Zustand drehfest verbunden sind, verdrehgesichert sind, so daß weitere Maßnahmen zur Verdrehsicherung der Stangen nicht erforderlich sind, wodurch der konstruktive Aufwand der erfindungsgemäßen Zange verringert wird.

In einer weiteren bevorzugten Ausgestaltung sind die Verbindungselemente der Stangen als Köpfe und die Verbindungselemente der Betätigungselemente als dazu im wesentlichen komplementäre Bohrungen ausgebildet, die an der distalen Stirnseite der Betätigungselemente und seitlich offen sind.

Durch diese Maßnahme wird eine konstruktiv besonders einfache Ausgestaltung der Verbindungselemente der Stangen sowie der Verbindungselemente der Betätigungselemente geschaffen, die im verbundenen Zustand außerdem einen mechanisch beständigen Kraftschluß zwischen den Stangen und den Betätigungselementen gewährleistet. Die Köpfe können bevorzugt als Kugeln und die Bohrungen als Kugelpfannen ausgebildet sein. Selbstverständlich ist es auch möglich, die Köpfe an den Betätigungselementen und die Bohrungen an den Stangen vorzusehen.

Dabei ist es bevorzugt, wenn die Verbindungselemente der Betätigungselemente als seitliche Öffnungen aufweisende Kugelpfannen ausgebildet sind und die seitliche Öffnung der ersten Kugelpfanne in die entgegengesetzte Richtung weist wie die seitliche Öffnung der zweiten Kugelpfanne.

Diese Maßnahme ist insbesondere in Verbindung mit dem bereits erwähnten Verbindungsmechanismus von Vorteil, bei dem die Verbindungselemente der Stangen mit den Verbindungselementen der Betätigungselemente durch Verschwenken der Stangen um die Längsachse in bzw. außer Eingriff gebracht werden. Diese Ausgestaltung ermöglicht es aber auch, die Verbindungselemente der Stangen mit den Verbindungselementen der Betätigungselemente durch axiales Zusammenschieben in Eingriff zu bringen.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf die Figuren der Zeichnung näher beschrieben. Es zeigen:
- Fig. 1: eine perspektivische Gesamtdarstellung einer erfindungsgemäßen Zange;
- Fig. 2: die Zange in Fig. 1 unter Wegiassung des Gehäuses;
- Fig. 3: eine der Fig. 2 entsprechende Darstellung der Zange, bei der die Stangen von den Betätigungselementen durch Verschwenken der Stangen gelöst sind;
- Fig. 4: die Baueinheit aus Stangen und Maulteilen der Zange in Fig. 1 in von der Handhabe abgenommenen Zustand;
- Fig. 5: den Schaft der Zange in Fig. 1 in von der Handhabe abgenommenen Zustand;
- Fig. 6: den Rastmechanismus der Zange in Fig. 1 zur Verbindung des Schaftes mit der Handhabe;
- Fig. 7a) und b): den distalen Abschnitt der Betätigungselemente und den proximalen Abschnitt der Stangen in vergrößertem Maßstab in zwei Teilbildern, die die Verbindung zwischen den Stangen und den Betätigungselementen zeigen; und
- Fig. 8a) und b): den proximalen Abschnitt der Stangen und den distalen Abschnitt der Betätigungselemente in vergrößertem Maßstab in zwei Teilbildern, die das Verbinden der Stangen mit den Betätigungselementen in einer alternativen Art und Weise zeigen.

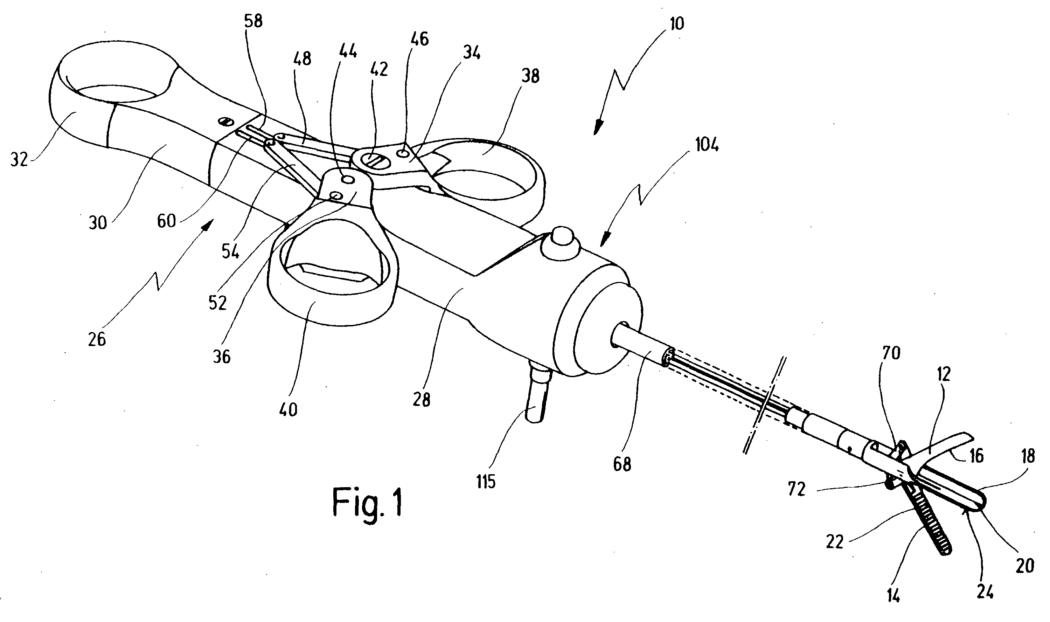

In Fig. 1 bis 3 ist eine mit dem allgemeinen Bezugszeichen 10 versehene medizinische Zange dargestellt. Die Zange 10 weist an ihrem distalen Ende ein erstes Maulteil 12 und ein zweites Maulteil 14 auf. Das erste Maulteil 12 und das zweite Maulteil 14 sind unabhängig voneinander beweglich, d.h. sie können unabhängig voneinander geöffnet bzw. geschlossen werden.

Das erste Maulteil 12 hat eine Schneidfunktion und ist dazu mit einer Schneide 16 ausgebildet, die beim Schließen des Maulteils 12 mit einer Schneide 18 eines unbeweglichen Maulbasisteils 20 in der Art einer Schere zusammenwirkt. Mit dem Maulteil 12 kann somit im menschlichen oder tierischen Körper Gewebe schneidend abgetrennt werden.

Das zweite Maulteil 14 ist als Faßwerkzeug ausgebildet und weist dazu eine gezahnte Fläche 22 auf, die mit einer an dem unbeweglichen Maulbasisteil 20 ausgebildeten gezahnten Fläche 24 zum Fassen von Gewebe zusammenwirkt.

Die medizinische Zange 10 wird bei einem endoskopischen chirurgischen Eingriff somit zum Schneiden von Gewebe sowie zum Fassen des abgeschnittenen Gewebes verwendet, um das abgetrennte Gewebe aus dem Körper zu entfernen. Es versteht sich jedoch, daß auch andere Maulteile mit anderen Funktionen im Rahmen der Erfindung verwendbar sind.

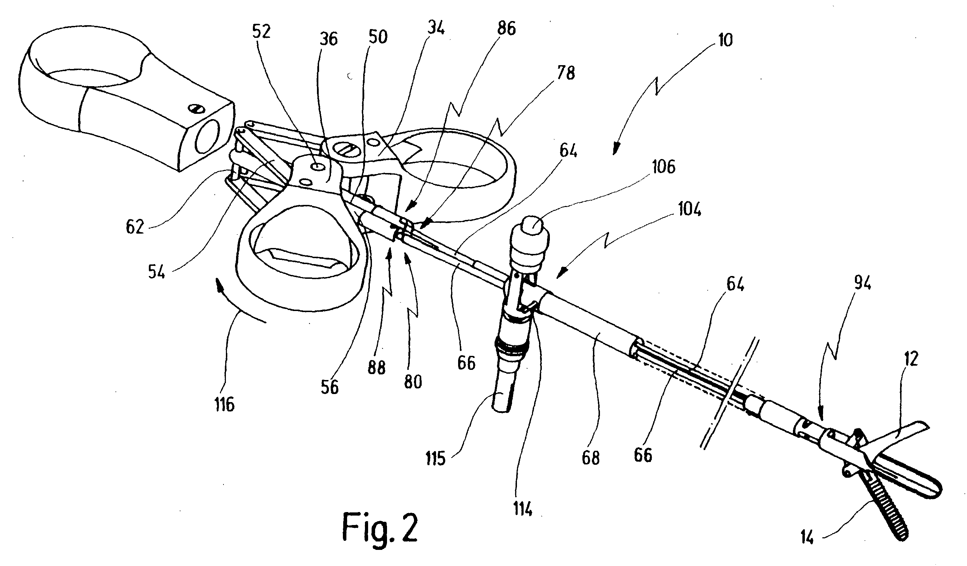

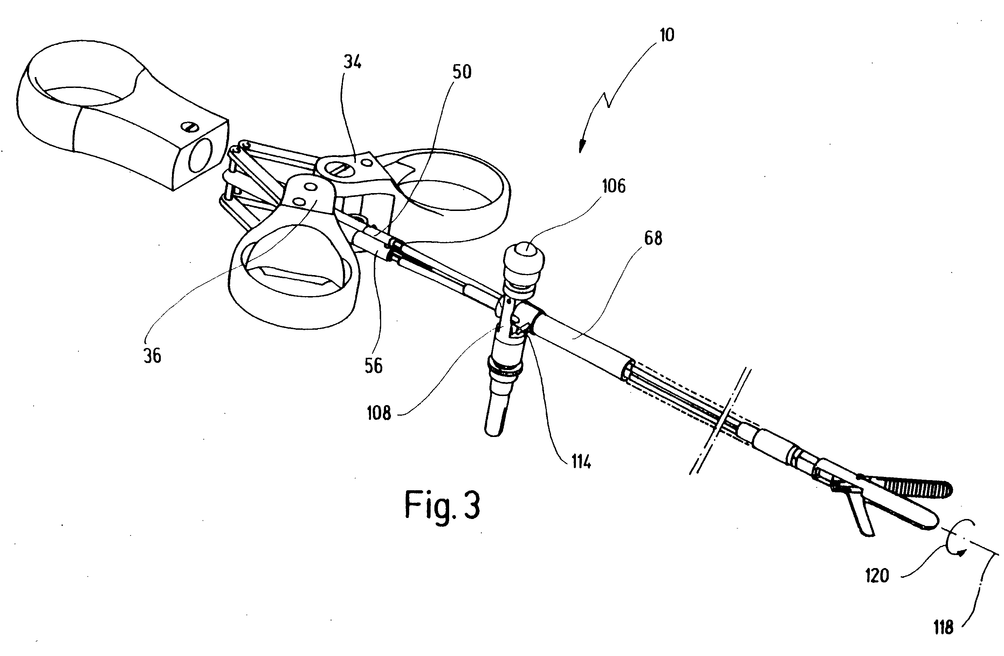

Am proximalen Ende weist die Zange 10 eine Handhabe 26 auf, die ein Gehäuse 28 aufweist, das in Fig. 2 und 3 nicht dargestellt ist. Am proximalen Ende der Handhabe 26 ist ein gehäusefestes unbewegliches Griffteil 30 angeordnet, das einen Daumenring 32 aufweist, durch den der Chirurg den Daumen einer Hand zur Bedienung der Zange 10 durchstecken kann.

Ferner weist die Handhabe 26 ein erstes bewegliches Griffteil 34 sowie ein zweites bewegliches Griffteil 36 auf. Das erste bewegliche Griffteil 34 dient der Betätigung des ersten Maulteiles 12, während das zweite bewegliche Griffteil 36 der Betätigung des zweiten Maulteiles 14 dient. Die beweglichen Griffteile 34 und 36 sind demnach unabhängig voneinander beweglich. Das erste bewegliche Griffteil 34 weist einen Fingerring 38 und das zweite bewegliche Griffteil 36 weist einen Fingerring 40 auf, durch die der die Zange 10 bedienende Chirurg den Zeigefinger derselben Hand, deren Daumen er in den Daumenring 32 steckt, durchstecken kann, um die Maulteile 12 bzw. 14 zu betätigen.

Das erste bewegliche Griffteil 34 ist über ein Scharniergelenk 42 und das zweite beweglich Griffteil 36 ist über ein Scharniergelenk 44 verschwenkbar an dem Gehäuse 28 befestigt.

An das erste beweglich Griffteil 34 ist bei 46 ein Hebelgestänge 48 angelenkt, wobei das Hebelgestänge 48 mit seinem proximalen Ende an einem ersten Betätigungselement 50 angelenkt ist, das in dem Gehäuse 28 angeordnet ist. Das erste Betätigungselement 50 ist in dem Gehäuse 28 axial verschiebbar. Entsprechend ist an dem zweiten beweglichen Griffteil 36 bei 52 ein weiteres Hebelgestänge 54 angelenkt, dessen proximales Ende wiederum an einem zweiten Betätigungselement 56 angelenkt ist, das in dem Gehäuse 28 parallel zu dem Betätigungselement 50 angeordnet und ebenfalls axial beweglich ist, wobei die Betätigungselemente 50 und 56 wiederum unabhängig voneinander axial beweglich sind. In dem Gehäuse 28 sind an dessem proximalen Ende ferner zwei Schlitze 58 und 60 ausgebildet, die als Führungsschlitze für Querstifte 62 der Hebelgestänge 48 und 54 dienen, die durch das proximale Ende der Betätigungselemente 50 und 56 durchgeführt sind.

Das erste Betätigungselement 50 ist mit seinem distalen Ende mit dem proximalen Ende einer ersten Stange 64 kraftschlüssig verbunden. Das zweite Betätigungselement 56 ist mit seinem distalen Ende mit dem proximalen Ende einer zweiten Stange 66 ebenfalls kraftschlüssig verbunden. Die Stangen 64 und 66 sind im Querschnitt rund.

Die erste Stange 64 und die zweite Stange 66 erstrecken sich längs nebeneinander liegend in einem Schaft 68 bis zum distalen Ende der Zange 10, an dem die erste Stange 64 über eine Kniehebelanordnung 70 mit dem ersten Maulteil 12 und die zweite Stange 66 über eine Kniehebelanordnung 72 mit dem zweiten Maulteil 14 kraftschlüssig verbunden sind.

Die erste Stange 64 und die zweite Stange 66 sind wie das erste Betätigungselement 50 und das zweite Betätigungselement 56 unabhängig voneinander axial verschiebbar in dem Schaft 68 angeordnet.

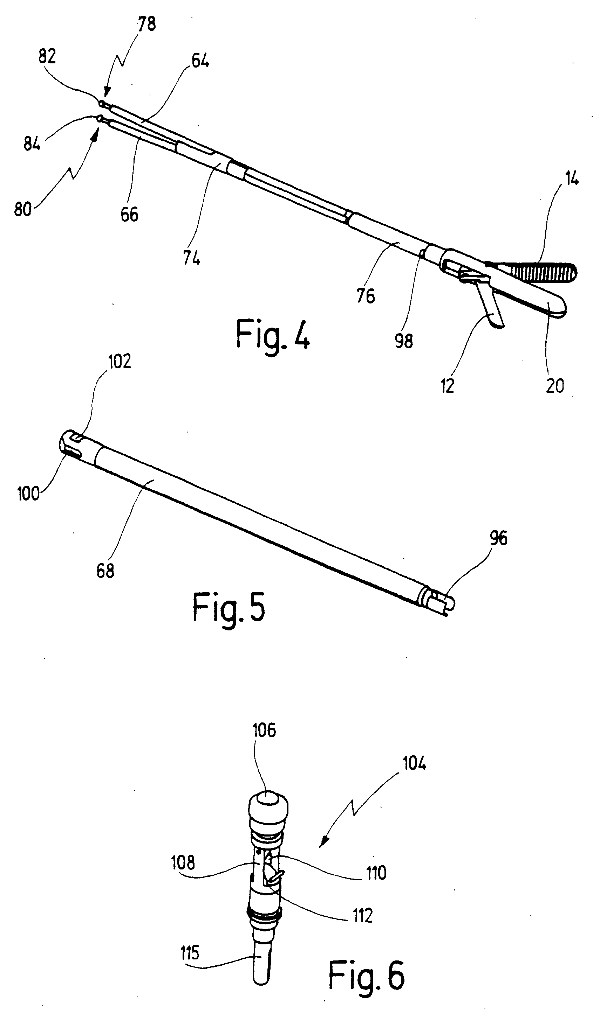

Die erste Stange 64 und die zweite Stange 66 sind mit dem ersten Betätigungselement 50 bzw. dem zweiten Betätigungselement 56 lösbar verbunden. Ebenso ist der Schaft 68 von der Handhabe 26 abnehmbar. In Fig. 4 sind die erste Stange 64 und die zweite Stange 66 zusammen mit den Maulteilen 12 und 14 im von der Handhabe 26 abgenommenen Zustand dargestellt, während in Fig. 5 der Schaft 68 ebenfalls in Alleinstellung dargestellt ist.

Gemäß Fig. 4 bilden die erste Stange 64 und die zweite Stange 66 zusammen mit den Maulteilen 12 und 14 eine Baueinheit. Die erste Stange 64 und die zweite Stange 66 verlaufen zwischen einer die beiden Stangen 64 und 66 umgebenden Hülse 74, in der die Stangen 64 und 66 verschiebbar durchgeführt sind, und einer an dem unbeweglichen Maulbasisteil 20 proximal sitzenden Hülse 76 parallel zueinander. Von der Hülse 74 bis zu ihrem proximalen Ende sind die erste Stange 64 und die zweite Stange 66 geringfügig voneinander abgespreizt. Weiterhin sind die erste Stange 64 und die zweite Stange 66 in dem zuvor genannten proximalen Bereich geringfügig elastisch federnd biegsam.

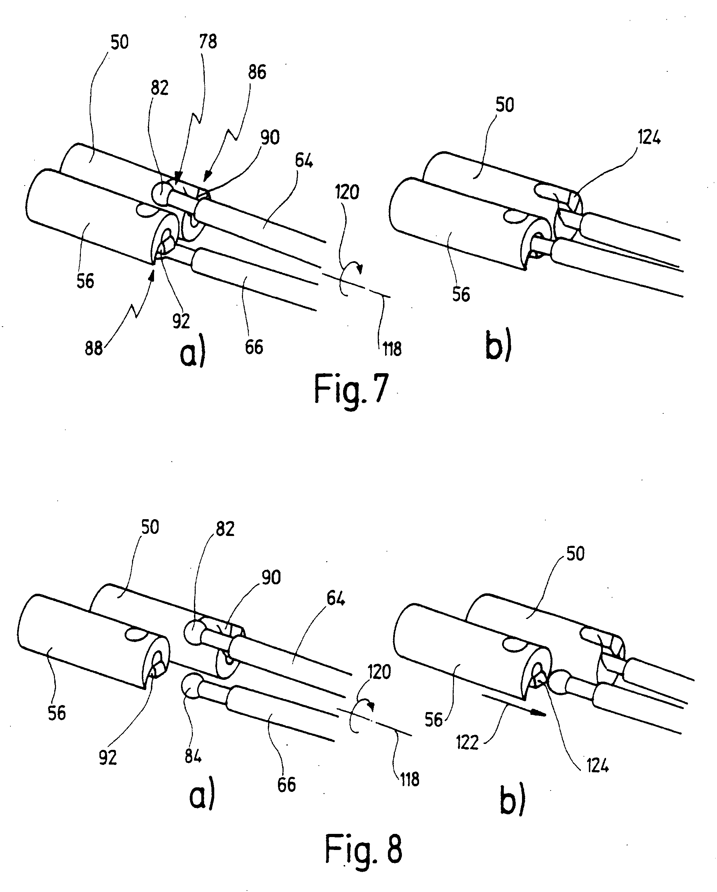

An ihrem proximalen Ende weist die erste Stange 64 ein Verbindungselement 78 und die zweite Stange 66 ein Verbindungselement 80 auf. Die Verbindungselemente 78 und 80 sind als Köpfe in Form von Kugeln 82 und 84 ausgebildet. Die Verbindungselemente 78 und 80 dienen zur kraftschlüssigen Verbindung der Stangen 64 und 66 mit den Betätigungselementen 50 und 56, die entsprechende, zu den Verbindungselementen 78 und 80 im wesentlichen komplementär ausgestaltete Verbindungselemente 86 und 88 aufweisen, die Bohrungen in Form von Kugelpfannen 90 bzw. 92 aufweisen (vergleiche insbesondere Fig. 7 und 8). Der Verbindungsmechanismus zwischen den Stangen 64 und 66 einerseits und den Betätigungselementen 50 und 56 andererseits wird an späterer Stelle noch näher beschrieben.

Die aus der ersten Stange 64, der zweiten Stange 66 und den Maulteilen 12 und 14 gebildete Baueinheit ist von dem Schaft 68 abnehmbar. Im zusammengesetzten Zustand ist die vorgenannte Baueinheit mit dem Schaft 68 mittels eines Bajonettverschlusses 94 (vergleiche Fig. 2) axial unverschiebbar verbunden, wobei aber die Stangen 64 und 166 relativ zu dem Schaft 68 verschiebbar sind. Der Bajonettverschluß 94 wird durch einen am distalen Ende des Schaftes 68 ausgebildeten Führungsschlitz 96 gebildet, der einen axial verlaufenden und einen umfänglichen Abschnitt aufweist, und in den ein an der Hülse 76 ausgebildeter Vorsprung 98 (vergleiche Fig. 4) eingreift.

Der Schaft 68 weist an seinem proximalen Ende eine Nut 100 auf, die einen in Fig. 5 sichtbaren axial verlaufenden Abschnitt und einen in Fig. 5 nicht sichtbaren umfänglich verlaufenden Abschnitt aufweist. Umfänglich zu dem axialen Abschnitt der Nut 100 versetzt weist der Schaft 68 eine etwa rechteckige sekantenartige Einkerbung 102 auf. Die Nut 100 und die Einkerbung 102 dienen zur Verrastung des Schaftes 68 an der Handhabe 26 mittels eines Rastmechanismus 104 (vergleiche Fig. 2). Der Rastmechanismus 104 ist mit dem Gehäuse 28 der Handhabe 26 fest verbunden.

In Fig. 6 ist der Rastmechanismus 104 in Alleinstellung dargestellt. Der Rastmechanismus 104 umfaßt einen Druckknopf 106, der mit einem Schieber 108 verbunden ist, so daß durch Herunterdrücken des Druckknopfes 106 auch der Schieber 108 nach unten gedrückt wird, wie in Fig. 3 dargestellt ist. Das Herunterdrücken des Druckknopfes 106 erfolgt gegen die Kraft einer Feder, die den Druckknopf 106 und damit den Schieber 108 in die in Fig. 2 dargestellte Lage nach oben drückt.

Der Schieber 108 weist mittig eine Öffnung 110 auf, in die das proximale Ende des Schaftes 68 gemäß Fig. 2 bzw. gemäß Fig. 3 durchgeführt ist. Die Öffnung 110 ist im oberen Bereich rund ausgebildet, weist im unteren Bereich jedoch einen radial nach innen ragenden Vorsprung 112 auf, der dazu vorgesehen ist, in die Einkerbung 102 des Schaftes 68 einzugreifen, um den Schaft 68 an der Handhabe 26 drehfest und axial unverschiebbar zu verrasten, wodurch auch die Baueinheit aus der erstan Stange 64, der zweiten Stange 66 und den Maulteilen 12 und 14 drehfest und axial unverschiebbar an der Handhabe 26 verrastet wird.

Der Rastmechanismus 104 umfaßt ferner einen an der Innenseite des Gehäuses 28 befestigten Stift 114, der in die Nut 100 des Schaftes 68 eingreift. Dem Druckknopf 106 gegenüberliegend ist noch ein Anschluß 115 zum Anschließen eines Hochfrequenzstromkabels angeordnet.

Die Maulteile 12 und/oder 14 können somit mit monopolarem oder bipolarem Hochfrequenzstrom beaufschlagt werden. Es ist jedoch auch möglich, die Zange 10 stromlos zu betreiben, oder den erfindungsgemäßen Verbindungsmechanismus zwischen den Stangen 64 und 66 und den Betätigungselementen 50 und 66 bei Vorsehung entsprechender Isolierungsmaßnahmen bei einer Zange für bipolaren Betrieb anzuwenden.

Ausgehend von Fig. 2, die den betriebsbereiten Zustand der Zange 10 (wobei das Gehäuse 28 zur Verdeutlichung weggelassen ist) darstellt, wird im folgenden die Funktion der Zange 10 sowie das Zerlegen der Zange 10 und das Zusammensetzen der Zange 10 näher beschrieben.

In Fig. 2 sind das bewegliche Griffteil 34 und das bewegliche Griffteil 36 in ihrer maximal distalen Schwenklage dargestellt. Entsprechend dieser Position der Griffteile 34 und 36 befinden sich das erste Betätigungselement 50 und das zweite Betätigungselement 56 und damit auch die erste Stange 64 und die zweite Stange 66 in ihrer maximal distalen Position, so daß das erste Maulteil 12 und das zweite Maulteil 14 ihre Offenstellung einnehmen. Wird nun beispielsweise das erste Griffteil 36 um das Scharniergelenk 44 gemäß einem Pfeil 116 in Richtung proximales Ende verschwenkt, so wird durch diese Schwenkbewegung das Hebelgestänge 54 nach proximal verschoben, wodurch das zweite Betätigungselement 56 und die zweite Stange 66 nach proximal gezogen werden und das zweite Maulteil 14 geschlossen wird. Entsprechend kann das erste Maulteil 12 durch Verschwenken des ersten beweglichen Griffteiles 34 nach proximal geschlossen werden.

Zum Zerlegen der Zange 10 wird zunächst der Druckknopf 106 nach unten gedrückt, um den Rastmechanismus 104 zu entriegeln. Durch Herunterdrücken des Druckknopfes 106 wird der Schieber 108 nach unten verschoben, wodurch der Vorsprung 112 mit der Einkerbung 102 an dem Schaft 68 außer Eingriff kommt. Der Schaft 68 läßt sich nun in der Öffnung 110 drehen, und zwar in der durch den umfänglichen Abschnitt der Nut 100, in die der Stift 114 eingreift, vorgegebenen Richtung.

Durch Drehen des Schaftes 68 um seine Mittelachse werden nun die erste Stange 64 und die zweite Stange 66 um eine Längsachse 118 gemäß einem Pfeil 120 verschwenkt, wodurch die Kugeln 82 und 84 am proximalen Ende der Stangen 64 und 66 mit den Kugelpfannen 90 und 92 am distalen Ende der Betätigungselemente 50 und 56 außer Eingriff kommen. Dazu sind die Kugelpfannen 90 und 92 seitlich offen, und zwar ist die seitliche Öffnung der Kugelpfanne 90 der seitlichen Öffnung der Kugelpfanne 92 diametral gegenüberliegend angeordnet. Die Verdrehung des Schaftes 68 um die Längsachse 118 ist auf einen Drehwinkel von etwa 90° begrenzt, da sich der umfängliche Abschnitt der Nut 100 des Schaftes 68 über einen Umfangswinkel von etwa 90° erstreckt.

Nach dem Verdrehen des Schaftes 68 und dem damit bewirkten Verschwenken der Stangen 64 und 66, die mit dem Schaft 68 noch drehfest verbunden sind, lassen sich der Schaft 68 und die Stangen 64 und 66 von der Handhabe 26 abnehmen, d.h. genauer gesagt, aus dem Gehäuse 28 herausziehen. Im nächsten Schritt kann noch der Schaft 68 von den Stangen 64 und 66 abgezogen werden, indem der Schaft 68 gegenüber den Stangen 64 und 66 zum Lösen des Bajonettverschlusses 94 verdreht wird. Das Verdrehen des Schaftes 68 und das damit verbundene Verschwenken der Stangen 64 und 66, durch das die Verbindungselemente 78 und 80 von den Verbindungselementen 86 und 88 getrennt werden, ist bei gedrücktem Druckknopf 106 unabhängig davon möglich, in welcher axialen Position sich die Betätigungselemente 50 und 56 gerade befinden, auch wenn dies in Fig. 3 bei maximal nach distal verschobenen Betätigungselementen 50 und 56 dargestellt ist.

Der Bajonettverschluß 941äßt sich nur lösen, wenn der Schaft 68 mit den Stangen 64 und 66 von der Handhabe abgenommen ist, indem die Verdrehrichtung zum Öffnen des Bajonettverschlusses der Verdrehrichtung zum Ausrücken der Kugeln 82 und 84 aus den Kugelpfannen 90 und 92 entgegengesetzt ist.

Zum Verbinden des Schaftes 68 und der Stangen 64 und 66 mit der Handhabe 26 wird in zu dem Zerlegevorgang umgekehrter Reihenfolge vorgegangen.

Zuerst werden die Stangen 64 und 66 in den Schaft 68 geschoben, bis die proximalen Enden der Stangen 64 und 66 aus dem proximalen Ende des Schaftes 68 vorragen, und der Schaft 68 wird mittels des Bajonettverschlusses 94 an der Hülse 76 befestigt. Anschließend wird die Anordnung aus dem Schaft 68 und den Stangen 64 und 66 in die distale Öffnung des Gehäuses 26 geschoben. Der gehäusefeste Stift 114 sorgt im Zusammenwirken mit dem axialen Abschnitt der Nut 100 des Schaftes 68 dafür, daß der Schaft 68 und damit die Stangen 64 und 66 nur in einer bestimmten Drehorientierung in das Gehäuse 26 eingeführt werden können. Beim Durchführen des proximalen Endes des Schaftes 68 durch die Öffnung 110 des Rastmechanismus 104 wird der Druckknopf 106 automatisch niedergedrückt. Die Einschubtiefe des Schaftes 68 in die Öffnung 110 wird durch das distale Ende des axialen Abschnittes der Nut 100 in dem Schaft 68 begrenzt.

Nachdem der Schaft 68 mit den Stangen 64 und 66 somit maximal in das Gehäuse 28 eingeschoben ist, befinden sich die Kugeln 82 und 84 der Stangen 64 und 66 in der in Fig. 7a) dargestellten Drehlage bezüglich der Kugelpfannen 90 und 92 der Betätigungselemente 50 und 56. Die in Fig. 7a) dargestellte Stellung, in der die Kugeln 82 und 84 auf gleicher axialer Höhe liegen wie die Kugelpfannen 90 und 92, ist beispielsweise dann gegeben, wenn die Betätigungselemente 50 und 56 beide in ihre maximal distale Position und auch die Stangen 64 und 66 in ihre maximal distale Stellung verschoben sind. In diesem Fall kommen die Kugeln 82 und 84 mit den Kugelpfannen 90 und 92 durch Verschwenken der Stangen 64 und 66 um die Längsachse 118 in Richtung des Pfeiles 120 in Eingriff und sind, wie in Fig. 7b) dargestellt ist, anschließend kraftschlüssig miteinander verbunden. Die Spreizung der distalen Enden der Stangen 64 und 66 dient dazu, daß der seitliche Abstand der Kugeln 82 und 84 dem seitlichen Abstand der Kugelpfannen 90 und 92 entspricht.

Das Verbinden der Stangen 64 und 66 mit den Betätigungselementen 50 und 56 ist jedoch auch dann möglich, wenn nach dem maximalen Einschieben des Schaftes 68 in das Gehäuse 28 die Kugel 82 und/oder die Kugel 84 sich nicht auf gleicher axialer Höhe befinden wie die Kugelpfanne 90 und/oder die Kugelpfanne 92, wie in Fig. 8a) für den Fall der Kugel 84 und die Kugelpfanne 92 dargestellt ist. Ein solcher Fall kann beispielsweise dann auftreten, wenn sich das Betätigungselement 56 nicht in seiner maximal distalen Position befindet, während sich die Stange 66 in ihrer maximal distalen Position befindet. In diesem Fall kommt bei einer Verschwenkung der Stangen 64 und 66 um die Längsachse 118 gemäß dem Pfeil 120 nur die Kugel 82 mit der Pfanne 90 in Eingriff, während die Kugel 84 distal vor der Kugelpfanne 92 zu liegen kommt, wie in Fig. 8b) dargestellt ist.

Das Betätigungselement 56 wird mit der Stange 66 in diesem Fall nun dadurch verbunden, daß das Betätigungselement 56 über das bewegliche Griffteil 36 in Richtung distales Ende gemäß einem Pfeil 122 verschoben wird. Die Kugelpfanne 92 weist ebenso wie die Kugelpfanne 90 eine stirnseitige Anlaufschräge 124 auf, wodurch die Kugel 84 beim Verschieben des Betätigungselementes 56 in Richtung des Pfeiles 122 auf die Anlaufschräge 124 aufläuft. Dabei wird das proximale Ende der Stange 66 aufgrund der elastischen Verbiegsamkeit aus seiner ursprünglichen Lage quer zu seiner Längsachse geringfügig ausgelenkt, und die Kugel 84 rastet anschließend in die Kugelpfanne 92 elastisch ein. Somit ist dann auch die Stange 66 mit dem Betätigungselement 56 kraftschlüssig verbunden.

Die Verbindung der Stangen 64 und 66 mit den Betätigungselementen 50 und 56 ist auch dann möglich, wenn sich beide Kugeln 82 und 84 nach dem Verschwenken der Stangen 64 und 66 distal vor den Kugelpfannen 90 und 92 befinden, wobei dann beide Betätigungselemente 50 und 56 in Richtung des Pfeiles 122 zur Herstellung der kraftschlüssigen Verbindung mit den Stangen 64 und 66 verschoben werden. Es kann aber auch der Fall auftreten, daß beim Einschieben des Schaftes 68 in das Gehäuse 26 sich die Kugeln 82 und 84 proximal hinter den Gelenkpfannen 90 und 92 befinden. In diesem Fall ist es nicht möglich, die Stangen 64 und 66 vollständig über 90° um die Längsachse 118 zu verschwenken. In diesem Fall werden die Griffteile 34 und 36 beide in Richtung proximales Ende verschwenkt, wodurch die Betätigungselemente 50 und 56 in ihre maximal proximale Lage zurückgezogen werden, so daß sich die Kugeln 82 und 84 der Stangen 64 und 66 auf axial gleicher Höhe oder distal vor den Kugelpfannen 90 und 92 befinden. Nun können die Stangen 64 und 66 um die Längsachse 118 gemäß dem Pfeil 120 vollständig verschwenkt werden, wonach die Betätigungselemente 34 und 36 wieder in Richtung distales Ende verschoben werden, wodurch, wie zuvor beschrieben, die Kugeln 82 und 84 über die Anlaufschrägen 124 in die Kugelpfannen 90 und 92 einrasten.

Während die erfindungsgemäße Zange 10 in Fig. 2 und 3, anhand derer das Verbinden der Stangen 64 und 66 mit den Betätigungselementen 50 und 56 beschrieben wurde, ohne das Gehäuse 28 dargestellt ist, versteht es sich von selbst, daß das Gehäuse sowohl beim Verbinden der Stangen 64 und 66 mit als auch beim Trennen der Stangen 64 und 66 von den Betätigungselementen 50 und 56 an der Handhabe 26 montiert ist. Sowohl das Lösen der Stangen 64 und 66 von den Betätigungselementen 50 und 56 als auch das Verbinden erfordert keine Sichtkontrolle oder sonstige Eingriffe von außen.

## Patentansprüche

1. Medizinische Zange, mit zwei unabhängig voneinander beweglichen Maulteilen (12, 14) am distalen Ende, mit einem Schaft (68), in dem sich zwei unabhängig voneinander axial verschiebbare, nebeneinander angeordnete Stangen (64, 66) längs erstrecken, wobei die erste Stange (64) mit dem ersten Maulteil (12) und die zweite Stange (66) mit dem zweiten Maulteil (14) kraftschlüssig verbunden ist, und mit einer Handhabe (26) am proximalen Ende, die zwei Betätigungselemente (50, 56) aufweist, wobei das erste Betätigungselement (50) mit der ersten Stange (64) und das zweite Betätigungselement (56) mit der zweiten Stange (66) kraftschlüssig verbunden ist, wobei die erste Stange (64) und die zweite Stange (66) an ihrem proximalen Ende jeweils ein Verbindungselement (78, 80) und das erste Betätigungselement (50) und das zweite Betätigungselement (56) an ihrem distalen Ende jeweils ein dazu im wesentlichen komplementär ausgebildetes Verbindungselement (86, 88) aufweisen, **dadurch gekennzeichnet, daß** die Verbindungselemente (78, 80) der Stangen (64, 66) mit den Verbindungselementen (86, 88) der Betätigungselemente (50, 56) durch axiales Zusammenschieben der Stangen (64, 66) und der Betätigungselemente (50, 56) und/oder durch eine gemeinsame Verschwenkung der beiden Stangen (64, 66) um eine Längsachse (118) kraftschlüssig, jedoch lösbar in Eingriff bringbar sind.

2. Zange nach Anspruch 1, **dadurch gekennzeichnet, daß** die Verbindungselemente (78, 80) der Stangen (64, 66) und die Verbindungselemente (86, 88) der Betätigungselemente (50, 56) derart ausgebildet sind, daß sie durch eine Verschwenkung der Stangen (64, 66) um die Längsachse (118) außer Eingriff bringbar sind.

3. Zange nach Anspruch 1 oder 2, **dadurch gekennzeichnet, daß** die Verbindungselemente (78, 80) der Stangen (64, 66) mit den Verbindungselementen (86, 88) der Betätigungselemente (50, 56) durch Betätigen der Betätigungselemente (50, 56) oder durch Betätigen der Maulteile (12, 14) rastend in Eingriff kommen.

4. Zange nach einem der Ansprüche 1 bis 3, **dadurch gekennzeichnet, daß** die Verbindungselemente (78, 80) der Stangen (64, 66) und/oder die Verbindungselemente (86, 88) der Betätigungselemente (50, 56) Anlaufschrägen (124) aufweisen.

5. Zange nach Anspruch 4, **dadurch gekennzeichnet, daß** die Stangen (64, 66) an ihrem proximalen Ende geringfügig elastisch federnd verbiegsam sind.

6. Zange nach einem der Ansprüche 1 bis 5, **dadurch gekennzeichnet, daß** die Stangen (64, 66) gegen unerwünschtes Verschwenken gesperrt sind, wenn die Verbindungselemente (78, 80) der Stangen (64, 66) mit den Verbindungselementen (86, 88) der Betätigungselemente (50, 56) in Eingriff stehen.

7. Zange nach einem der Ansprüche 1 bis 6, **dadurch gekennzeichnet, daß** der Schaft (68) abnehmbar mit der Handhabe (26) verbunden ist.

8. Zange nach Anspruch 7, **dadurch gekennzeichnet, daß** der Schaft (68) mittels eines an der Handhabe (26) angeordneten Rastmechanismus (104) mit der Handhabe (26) verbunden ist.

9. Zange nach Anspruch 8, **dadurch gekennzeichnet, daß** der Rastmechanismus (104) den Schaft (68) gegen Verdrehen sperrt.

10. Zange nach einem der Ansprüche 1 bis 9, **dadurch gekennzeichnet, daß** der Schaft (68) von den Stangen (64, 66) abnehmbar ist, und daß die Stangen (64, 66) mit dem Schaft (68) im montierten Zustand verbunden sind.

11. Zange nach einem der Ansprüche 1 bis 10, **dadurch gekennzeichnet, daß** die Verbindungselemente (78, 80) der Stangen (64, 66) als Köpfe und die Verbindungselemente (86, 88) der Betätigungselemente (50, 56) als dazu im wesentlichen komplementäre Bohrungen ausgebildet sind, die an der distalen Stirnseite der Betätigungselemente (50, 56) und seitlich offen sind, oder umgekehrt.

12. Zange nach Anspruch 11, **dadurch gekennzeichnet, daß** die Verbindungselemente (86, 88) der Betätigungselemente (50, 56) als seitliche Öffnungen aufweisende Kugelpfannen (90, 92) ausgebildet sind und die seitliche Öffnung der ersten Kugelpfanne (90) in die entgegengesetzte Richtung weist wie die seitliche Öffnung der zweiten Kugelfanne (92).

## Claims

1. A medical forceps comprising two independently moveable jaw parts (12, 14) at the distal end, a shaft (68) having two rods (64, 66) extending longitudinally therein, the rods being arranged adjacent to one another and being axially displaceable independent from one another, wherein the first rod (64) is force-lockingly connected with the first jaw part (12) and the second rod (66) is force-lockingly connected with the second jaw part (14), and a handle (26) at the proximal end having two actuator elements (50, 56), wherein the first actuator element is force-lockingly connected with the first rod (64) and the second actuator element (56) is force-lockingly connected with the second rod (66), wherein the first rod (64) and the second rod (66) each comprise a coupling element (78, 80) at their proximal ends, the first actuator element (50) and the second actuator element (56) each comprise a substantially complementary coupling element (86, 88) at their distal ends, **characterized in that** the coupling elements (78, 80) of the rods (64, 66) are engageable in force-locking, but releasable manner with the coupling elements (86, 88) of the actuator elements (50, 56) by axially pushing the rods (64, 66) and the actuator elements (50, 56) together and/or by a common rotation of the two rods (64, 66) about a longitudinal axis (118).

2. The forceps of claim 1, **characterized in that** the coupling elements (78, 80) of the rods (64, 66) and the coupling elements (86, 88) of the actuator elements (50, 56) are configured such that they are disengageable by a rotation of the rods (64, 66) about the longitudinal axis (118).

3. The forceps of claim 1 or 2, **characterized in that** the coupling elements (78, 80) of the rods (64, 66) become lockingly engaged with the coupling elements (86, 88) of the actuator elements (50, 56) by actuating the actuator elements (50, 56) or by actuating the jaw parts (12, 14).

4. The forceps of one of claims 1 through 3, **characterized in that** the coupling elements (78, 80) of the rods (64, 66) and/or the coupling elements (86, 88) of the actuator elements (50, 56) comprise engagement slopes (124).

5. The forceps of claim 4, **characterized in that** the rods (64, 66) are slightly elastically bendable at their proximal ends.

6. The forceps of one of claims 1 through 5, **characterized in that** the rods (64, 66) are restricted against undesired rotation when the coupling elements (78, 80) of the rods (64, 66) are engaged with the coupling elements (86, 88) of the actuator elements (50, 56).

7. The forceps of one of claims 1 through 6, **characterized in that** the shaft (68) is connected to the handle (26) in detachable manner.

8. The forceps of claim 7, **characterized in that** the shaft (68) is connected to the handle (26) by means of a locking mechanism (104) arranged on the handle (26).

9. The forceps of claim 8, **characterized in that** the locking mechanism (104) secures the shaft (68) against rotation.

10. The forceps of one of claims 1 through 9, **characterized in that** the shaft (68) is detachable from the rods (64, 66) and that the rods (64, 66) are connected to the shaft (68) in the assembled condition.

11. The forceps of one of claims 1 through 10, **characterized in that** the coupling elements (78, 80) of the rods (64, 66) are formed as heads and the coupling elements (86, 88) of the actuator elements (50, 56) are formed as substantially complementary bore holes, which are open on the distal end side of the actuator elements (50, 56) and are open to the side, or vice versa.

12. The forceps of claim 11, **characterized in that** the coupling elements (86, 88) of the actuator elements (50, 56) are configured as ball sockets (90, 92) comprising side openings, and the side opening of the first ball socket (90) is directed in the opposite direction with respect to the side opening of the second ball socket (92).

## Revendications

1. Pince médicale comportant deux parties de mâchoire (12, 14) déplaçables indépendamment l'une de l'autre à l'extrémité distale, comportant une tige (68) dans laquelle s'étendent longitudinalement deux tringles (64, 66) pouvant coulisser axialement indépendamment l'une de l'autre et disposées côte à côte, la première tringle (64) étant reliée à force à la première partie de mâchoire (12) et la deuxième tringle (66), à la deuxième partie de mâchoire (14), et comportant une poignée (26) à l'extrémité proximale, qui comporte deux éléments d'actionnement (50, 56), le premier élément d'actionnement (50) étant relié à force à la première tringle (64), le deuxième élément d'actionnement (56), à la deuxième tringle (66), la première tringle (64) et la deuxième tringle (66) comportant chacune à leur extrémité proximale un élément de liaison (78, 80) et le premier élément d'actionnement (50) ainsi que le deuxième élément d'actionnement (56) comportant chacun à leur extrémité distale un élément de liaison (86, 88) conformé de manière sensiblement complémentaire au précédent élément de liaison, **caractérisée en ce que** les éléments de liaison (78, 80) des tringles (64, 66) peuvent être amenés en engagement à force, mais séparable, avec les éléments de liaison (86, 88) des éléments d'actionnement (50, 56), par réunion par coulissement axial des tringles (64, 66) et des éléments d'actionnement (50, 56) et/ou par un pivotement commun des deux tringles (64, 66), autour d'un axe longitudinal (118).

2. Pince selon la revendication 1, **caractérisée en ce que** les éléments de liaison (78, 80) des tringles (64, 66) et les éléments de liaison (86, 88) des éléments d'actionnement (50, 56) sont conformés de manière à pouvoir être désengagés par un pivotement des tringles (64, 66) autour de l'axe longitudinal (118).

3. Pince selon la revendication 1 ou 2, **caractérisée en ce que** les éléments de liaison (78, 80) des tringles (64, 66) viennent en engagement par encliquetage avec les éléments de liaison (86, 88) des éléments d'actionnement (50, 56), par actionnement des éléments d'actionnement (50, 56) ou par actionnement des parties de mâchoire (12, 14).

4. Pince selon l'une des revendications 1 à 3, **caractérisée en ce que** les éléments de liaison (78, 80) des tringles (64, 66) et/ou les éléments de liaison (86, 88) des éléments d'actionnement (50, 56) présentent des chanfreins de montée (124).

5. Pince selon la revendication 4, **caractérisée en ce que** les tringles (64, 66) peuvent être pliées légèrement à leur extrémité proximale, de manière élastique.

6. Pince selon l'une des revendications 1 à 5, **caractérisée en ce que** les tringles (64, 66) sont bloquées contre un pivotement indésirable, lorsque les éléments de liaison (78, 80) des tringles (64, 66) sont en engagement avec les éléments de liaison (86, 88) des éléments d'actionnement (50, 56).

7. Pince selon l'une des revendications 1 à 6, **caractérisée en ce que** la tige (68) est reliée de manière amovible à la poignée (26).

8. Pince selon la revendication 7, **caractérisée en ce que** la tige (68) est reliée à la poignée (26) au moyen d'un mécanisme à encliquetage (114) disposé sur la poignée (26).

9. Pince selon la revendication 8, **caractérisée en ce que** le mécanisme d'encliquetage (104) bloque la tige (68) contre une rotation.

10. Pince selon l'une des revendications 1 à 9, **caractérisée en ce que** la tige (68) peut être enlevée des tringles (64, 66) et **en ce que** les tringles (64, 66) sont reliées à la tige (68) à l'état monté.

11. Pince selon l'une des revendications 1 à 10, **caractérisée en ce que** les éléments de liaison (78, 80) des tringles (64, 66) sont conformés en tant que têtes et les éléments de liaison (86, 88) des éléments d'actionnement (50, 56) en tant que trous sensiblement complémentaires à ces têtes, lesquels trous sont ouverts latéralement sur le côté frontal distal des éléments d'actionnement (50, 56) ou inversement.

12. Pince selon la revendication 11, **caractérisée en ce que** les éléments de liaison (86, 88) des éléments d'actionnement (50, 56) sont conformés en coussinets sphériques (88, 92), présentant des ouvertures latérales, et l'ouverture latérale du premier coussinet sphérique (90) est dirigée dans le sens opposé à l'ouverture latérale du deuxième coussinet sphérique (92).
